(19) Europäisches Patentamt
European Patent Office
Office européen des brevets

(11) Publication number: **0 527 720 B1**

# (12) EUROPEAN PATENT SPECIFICATION

(45) Date of publication of patent specification: **17.08.94**

(51) Int. Cl.5: **A61K 31/47**, A61K 31/55, //(A61K31/47,31:435,31:445, 31:41,31:40,31:185)

(21) Application number: **90904349.9**

(22) Date of filing: **12.03.90**

(86) International application number: **PCT/EP90/00430**

(87) International publication number: **WO 90/10445 (20.09.90 90/22)**

## (54) THERAPEUTIC AGENTS.

(30) Priority: **15.03.89 US 324213**

(43) Date of publication of application: **24.02.93 Bulletin 93/08**

(45) Publication of the grant of the patent: **17.08.94 Bulletin 94/33**

(84) Designated Contracting States: **AT BE CH DE DK ES FR GB IT LI LU NL SE**

(56) References cited:
**EP-A- 0 224 810**

**J.Hypertension, vol.4, uppl. 6, 1986, Gower Medical Publishing Ltd.,(London, GB), P.Kerth et al. pages 128-130**

(73) Proprietor: **The Boots Company PLC**
**1 Thane Road West**
**Nottingham Nottinghamshire NG2 3AA (GB)**

(72) Inventor: **O'CONNOR, Patrick, Coleman**
**522 Rock Hollow Drive**
**Shreveport, LA 71115 (US)**
Inventor: **DEFESCHE, Charles, Leon-Marie**
**282 Woodstone Circle**
**Buffalo Grove, IL 60089 (US)**

(74) Representative: **Filler, Wendy Anne et al**
**The Boots Company plc**
**Patents Department**
**R4 Pennyfoot Street**
**GB-Nottingham NG2 3AA (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

EP 0 527 720 B1

## Description

This invention relates to products and pharmaceutical compositions suitable for use in the treatment of heart failure. More particularly, the invention relates to products and compositions for the treatment of heart failure comprising a quinolone derivative and an angiotensin-converting enzyme (ACE) inhibitor.

Heart failure is a major health problem in humans causing inadequate blood circulation resulting in the death or disability of sufferers. Although many cases of heart failure have an ischaemic aetiology, other causes are, for example, valvular heart disease and primary or secondary cardiomyopathies.

US Patent No. 4552884 describes a method of treating heart failure in mammals by the administration of a quinolone derivative of formula

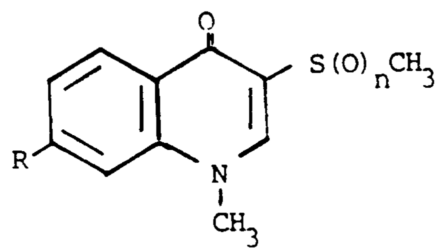

wherein n is 0, 1 or 2, and R is hydrogen, halogen, methyl or trifluoromethyl. The compounds are described as vasodilators, with a dilating action on both arteriolar and venous vascular beds.

Compounds are known which inhibit the activity of angiotensin-converting enzyme (ACE). This enzyme catalyses the conversion of the inactive decapeptide angiotensin I to the active octapeptide angiotensin II. Angiotensin II is a powerful vasoconstrictor and also stimulates aldosterone release from the adrenal glands. Aldosterone increases blood volume and hence blood pressure by promoting sodium and water retention by the kidneys. Thus compounds which inhibit ACE lower the level of angiotensin II in plasma and lead to a reduction of blood pressure and a reduction of preload and afterload of the heart. Such compounds, known as ACE inhibitors, are valuable therapeutic agents in the treatment of hypertension and heart failure and their use has been described for example by J.G.F. Cleland in Cardiology in Practice, 5 (3), 18-32 (1987)

It has now been found that administration of an ACE inhibitor and a particular type of quinolone derivative has certain advantages in the treatment of heart failure.

Accordingly, the present invention provides products and compositions for the treatment of heart failure in a human in need of such treatment which comprise a therapeutically effective amount of an angiotensin-converting enzyme (ACE) inhibitor and a therapeutically effective amount of a compound of formula I

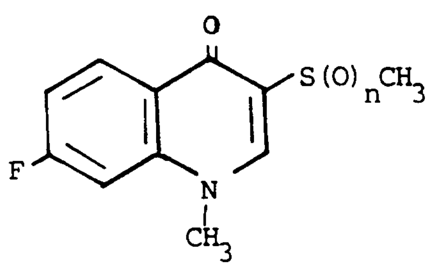

(I)

wherein n is 1 or 2 and wherein the active ingredients are provided for either concomitant or concurrent administration.

Suitable ACE inhibitors are known in the art and include, for example, captopril, enalapril, enalaprilat, quinapril, ramipril, cilazapril, delapril, fosenopril, zofenopril, indolapril, perindopril, spirapril, pentopril, pivopril, benazepril, benazeprilat, libenzapril and lisinopril. Preferred ACE inhibitors are captopril, enalapril and lisinopril. It will also be appreciated by those skilled in the art that where the ACE inhibitor is an acid or a base, it may be used in the form of a pharmaceutically acceptable base or acid addition salt thereof. The names indicated are International Non-proprietory Names (INN) or United States Adopted Names (USAN).

The particularly preferred compound of formula I, wherein n is 1, is 7-fluoro-1-methyl-3-methylsulphinyl-4-quinolone, the International Non-proprietory Name (INN) of which is flosequinan. The compound of formula I wherein n is 2, 7-fluoro-1-methyl-3-methylsulphonyl-4-quinolone, is the principal metabolite of

flosequinan in man and has been shown to have similar pharmacological properties as described for example by Wynne et al. in Eur. J. Clin. Pharmacol. (1985), 28, 659-664.

The products and compositions of the present invention may be used in the treatment of acute heart failure, for example occurring as a result of acute myocardial infarction. However, a preferred method of the present invention is the treatment of chronic heart failure, particularly congestive heart failure. Severe congestive heart failure is a disabling disease with a high mortality. The method according to the present invention gives beneficial effects in many patients suffering from chronic heart failure. Such beneficial effects may include, for example, increased exercise tolerance, enhanced quality of life in terms of fatigue and breathlessness and improved life expectancy.

Chronic congestive heart failure patients commonly show an inadequate response to ACE inhibitor therapy. This may be due to a poor or partial response to administration of the drug over a two to three month trial of therapy. Alternatively, an inadequate response may become apparent as the initial symptomatic improvements seen in many chronic heart failure patients being treated with an ACE inhibitor deteriorate after a period of several months or years. It is known that increasing the dose of an ACE inhibitor will not increase the magnitude of its effect and thus there is no virtue in giving larger doses to non-responders. The method according to the invention surprisingly produces excellent improvements in the haemodynamic parameters and the clinical symptoms of many congestive heart failure patients showing an inadequate response to therapy with ACE inhibitors, either as monotherapy or administered with concurrent diuretic treatment.

Many vasodilatory drugs are associated with reflex tachycardia and clinical studies have shown that an increase of heart rate occurs in heart failure patients receiving 150 mg doses of flosequinan. An increase of heart rate is generally an undesirable effect in the treatment of heart failure since additional stress is placed on the failing heart. Furthermore, patients receiving such therapy may report adverse drug events associated with the increase in heart rate such as tachycardia and palpitations. These undesirable side effects of vasodilatory drug therapy effectively limit the maximum dose of the drug which can be safely administered. Clinical studies which exemplify the method according to the invention suggest that such an increase of heart rate may be substantially controlled by co-administration of flosequinan with an ACE inhibitor. Hence, the method provides the possibility of administering dosages of a compound of formula I higher than those used in conventional monotherapy, thus producing greater vasodilatory effects and benefits derived therefrom.

The products and compositions of the present invention are formed so that the active ingredients may be administered concomitantly or concurrently, for example in the form of separate dosage units to be used simultaneously, separately or sequentially. Accordingly, the invention provides a product containing an angiotensin-converting enzyme (ACE) inhibitor and a compound of formula I

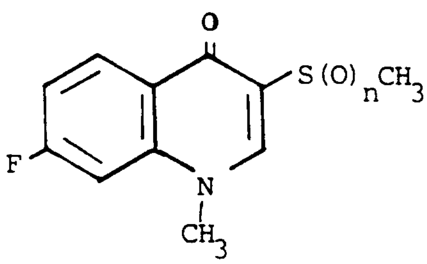

(I)

wherein n is 1 or 2, as a combined preparation for simultaneous, separate or sequential use for the treatment of heart failure.

The ratio of the ACE inhibitor to compound of formula I used is such that the quantity of each active ingredient employed will be such as to provide a therapeutically effective level but will not be larger than the quantity recommended as safe for administration.

The active ingredients may be administered orally, rectally, parenterally or topically in the form of one or more pharmaceutical compositions comprising one or both of the active compounds together with at least one pharmaceutically acceptable carrier. Oral administration is preferred for the treatment of chronic heart failure, but intravenous administration may be preferred for the treatment of the acute stage of this disease, for example, heart failure complicating acute myocardial infarction. The precise amount of each of the active compounds administered will depend on a number of factors including the age of the patient, the severity of the condition and the past medical history, among other factors, and always lies within the sound discretion of the administering physician.

In general, dosage of the compounds of formula I, especially flosequinan, as employed for an adult human treatment is within the range 1-500 mg/day when administered orally. Accordingly, suitably 10-300 mg/day is administered, more usually 10-200 mg/day, and especially 50-150 mg/day, given in single or divided doses. For parenteral administration, a suitable dose of flosequinan is generally within the range 1-300 mg/day and suitably 10-200 mg/day is administered in single or divided doses. The oral dosage of ACE inhibitor as employed for an adult human treatment is generally in the range from 2.5 to 150 mg/day. For example a suitable dosage of the ACE inhibitor captopril is 18-300 mg/day, preferably 18-100 mg/day and a suitable dosage of lisinopril or enalapril is 2.5-20 mg/day, administered in single or divided doses.

In utilising the products and compositions of the present invention it may be advantageous to include one or more compatible pharmacologically active ingredients, for example a diuretic such as furosemide, chlorthalidone or hydrochlorothiazide, a cardiac glycoside such as digitalis or digoxin or a nitrate compound such as glyceryl trinitrate.

In a further aspect of the present invention, there is provided a pharmaceutical composition suitable for use in the treatment of heart failure comprising therapeutically effective amounts of an ACE inhibitor and a compound of formula I as defined hereinbefore, together with a pharmaceutically acceptable carrier. Such pharmaceutical compositions may take the form of any of the known pharmaceutical compositions for oral, rectal, parenteral or topical administration and the pharmaceutically acceptable carriers suitable for use in such compositions are well known in the art of pharmacy. The compositions of the invention suitable contain 0.1-90% by weight of an ACE inhibitor and from 0.1-90% weight of the compound of formula I. The compositions of the invention are often prepared in unit dosage form.

Compositions for oral administration are the known pharmaceutical forms for such administration, for example tablets, capsules, granules, syrups and aqueous or oily suspensions. The excipients used in the preparation of these compounds are the excipients known in the pharmacist's art. Tablets may be prepared by mixing the active compounds with suitable excipients such as inert diluents, disintegrants, lubricants and binders, and tabletting the mixture by known methods. Diluents may include, for example, calcium phosphate and lactose. Disintegrants may include, for example, maize starch and croscarmellose sodium. Lubricants may include, for example, magnesium stearate and stearic acid. Binders may include, for example, microcrystalline cellulose and polyvinylpyrrolidone. Tablets may, if desired, be provided with enteric coatings by known methods, for example by the use of cellulose acetate phthalate. Similarly capsules, for example hard or soft gelatin capsules, containing the active compounds with or without added excipients, may be prepared by conventional means and, if desired, provided with enteric coatings in a known manner. The tablets and capsules may conveniently each contain 5-200 mg of a compound of formula I and 2.5-150 mg, preferably 2.5-50 mg of an ACE inhibitor. Other compositions for oral administration include, for example, aqueous suspensions containing the active compounds in an aqueous medium in the presence of a non-toxic suspending agent such as sodium carboxymethylcellulose, and oily suspensions containing the active compounds in a suitable vegetable oil, for example arachis oil.

Compositions of the invention suitable for rectal administration are the known pharmaceutical forms for such administration, for example, suppositories with cocoa butter or polyethylene glycol bases.

Compositions of the invention suitable for parenteral administration are the known pharmaceutical forms for such administration, for example, sterile suspensions in aqueous and oily media or sterile solutions in a suitable solvent.

Compositions for topical administration may comprise a matrix in which the active compounds are dispersed so that the compounds can be held in contact with the skin in order to administer the active compounds transdermally. Alternatively, the active compounds may be dispersed in a cream or ointment base.

In some formulations it may be beneficial to use the active compounds in the form of particles of very small size, for example, as obtained by fluid energy milling.

Especially valuable compositions for use in accordance with the present invention comprise flosequinan and an ACE inhibitor selected from captopril, enalapril and lisinopril. Enalapril and lisinopril are particularly preferred ACE inhibitors for use in the compositions according to the invention because of their compatibility with the once a day dosing of flosequinan.

In the compositions of the present invention the active compounds may, if desired, be associated with one or more other compatible pharmacologically active ingredients, for example a diuretic such as furosemide, chlorthalidone or hydrochlorothiazide or a cardiac glycoside such as digitalis or digoxin.

The following are examples of compositions which may be used in accordance with the method of the present invention.

Composition Example 1

In the preparation of capsules, 50 parts by weight of flosequinan, 50 parts by weight of captopril and 300 parts by weight of lactose are deaggregated and blended. The mixture is filled into hard gelatin capsules, each capsule containing 50 mg of flosequinan in 50 mg captopril.

Composition Example 2

In the preparation of capsules, 100 parts by weight of flosequinan, 50 parts by weight of captopril and 300 parts by weight of lactose are deaggregated and blended. The mixture is filled into hard gelatin capsules, each capsule containing 100 mg of flosequinan and 50 mg of captopril.

Composition Example 3

In the preparation of capsules, 100 parts by weight of flosequinan, 20 parts by weight of lisinopril and 280 parts by weight of lactose are deaggregated and blended. The mixture is filled into hard gelatin capsules, each capsule containing 100 mg of flosequinan and 20 mg of lisinopril.

Composition Example 4

In the preparation of capsules, 100 parts by weight of flosequinan, 5 parts by weight of enalapril maleate and 295 parts by weight of lactose are deaggregated and blended. The mixture is filled into hard gelatin capsules, each capsule containing 100 mg of flosequinan and 5 mg of enalapril maleate.

Composition Example 5

Tablets are prepared from the following ingredients:-

| | Parts by weight |
|---|---|
| Flosequinan | 50 |
| Captopril | 25 |
| Lactose | 100 |
| Microcrystalline cellulose | 50 |
| Croscarmellose sodium | 25 |
| Polyvinylpyrrolidone | 10 |
| Stearic acid | 2 |

The active ingredients, lactose, microcrystalline cellulose and croscarmellose sodium are deaggregated, blended and the resulting mixture is granulated with a solution of the polyvinylpyrrolidone in ethanol. The dry, sized granulate is blended with the stearic acid. The mixture is then compressed in a tabletting machine to give tablets containing 50 mg of flosequinan and 25 mg of captopril.

Composition Example 6

Tablets are prepared from the following ingredients:-

| | Parts by weight |
|---|---|
| Flosequinan | 150 |
| Enalapril maleate | 5 |
| Lactose | 75 |
| Microcrystalline cellulose | 40 |
| Croscarmellose sodium | 32 |
| Polyvinylpyrrolidone | 13 |
| Magnesium stearate | 3 |

The active ingredients, lactose, microcrystalline cellulose and croscarmellose sodium are deaggregated, blended and the resulting mixture is granulated with a solution of the polyvinylpyrrolidone in ethanol. The dry, sized granulate is blended with the magnesium stearate. The mixture is then compressed in a tabletting machine to give tablets containing 150 mg of flosequinan and 5 mg of enalapril maleate.

Composition Example 7

Tablets are prepared from the following ingredients:-

| | Parts by weight |
|---|---|
| Flosequinan | 150 |
| Lisinopril | 10 |
| Lactose | 70 |
| Microcrystalline cellulose | 40 |
| Croscarmellose sodium | 32 |
| Polyvinylpyrrolidone | 13 |
| Magnesium stearate | 3 |

The ingredients are formulated as described in Example 6 and compressed to give tablets containing 150 mg of flosequinan and 10 mg of lisinopril.

Composition Example 8

Tablets are prepared from the following ingredients:-

| | Parts by weight |
|---|---|
| Flosequinan | 100 |
| Lisinopril | 5 |
| Lactose | 115 |
| Microcrystalline cellulose | 60 |
| Croscarmellose sodium | 35 |
| Polyvinylpyrrolidone | 13 |
| Magnesium stearate | 3 |

The ingredients are formulated as described in Example 6 and compressed to give tablets containing 100 mg of flosequinan and 5 mg of lisinopril.

Composition Example 9

Tablets are prepared from the following ingredients:-

| | Parts by weight |
|---|---|
| Flosequinan | 150 |
| Lisinopril | 20 |
| Lactose | 60 |
| Microcrystalline cellulose | 40 |
| Croscarmellose sodium | 32 |
| Polyvinylpyrrolidone | 13 |
| Magnesium stearate | 3 |

The ingredients are formulated as described in Example 6 and compressed to give tablets containing 150 mg of flosequinan and 20 mg of lisinopril.

Composition Example 10

Tablets are prepared from the following ingredients:-

| | Parts by weight |
|---|---|
| Flosequinan | 100 |
| Enalapril maleate | 10 |
| Lactose | 110 |
| Microcrystalline cellulose | 60 |
| Croscarmellose sodium | 35 |
| Polyvinylpyrrolidone | 13 |
| Magnesium stearate | 3 |

The ingredients are formulated as described in Example 6 and compressed to give tablets containing 100 mg of flosequinan and 10 mg of enalapril maleate.

Composition Example 11

Tablets are prepared from the following ingredients:-

| | Parts by weight |
|---|---|
| Flosequinan | 150 |
| Enalapril maleate | 20 |
| Lactose | 60 |
| Microcrystalline cellulose | 40 |
| Croscarmellose sodium | 32 |
| Polyvinylpyrrolidone | 13 |
| Magnesium stearate | 3 |

The ingredients are formulated as described in Example 6 and compressed to give tablets containing 150 mg of flosequinan and 20 mg of enalapril maleate.

Composition Example 12

Tablets are prepared from the following ingredients:-

| | Parts by weight |
|---|---|
| Flosequinan | 100 |
| Captopril | 25 |
| Lactose | 150 |
| Microcrystalline cellulose | 75 |
| Croscarmellose sodium | 40 |
| Polyvinylpyrrolidone | 16 |
| Stearic acid | 3 |

The ingredients are formulated as described in Example 5 and compressed to give tablets containing 100 mg of flosequinan and 25 mg of captopril.

The following non-limitative Clinical Example illustrates the invention.

Clinical Example

Beneficial effects derived from administration of an ACE inhibitor with a quinolone of formula I have been demonstrated in a clinical study. Nine patients presented with chronic congestive heart failure which was not adequately controlled by a stable daily dosage of diuretics and an ACE inhibitor. The etiology of

the heart failure was either coronary artery disease (5 patients) or ischaemic heart disease (4 patients).

Patients had received a stable daily dose of captopril (average dose = 76.3 mg/day) for a minimum of three months and were showing an inadequate response to the treatment. They exhibited typical clinical symptoms of NYHA Class III and IV congestive heart failure such as breathlessness, fatigue and pulmonary oedema and had a mean left ventricular ejection fraction of 22.8%. Right heart and radial artery catheters were appropriately placed and baseline haemodynamic parameters were measured. Baseline pulmonary capillary wedge pressures (PCWP) greater than or equal to 15 mmHg on Day 1 were required for inclusion in the study.

The haemodynamic parameters of the patients were monitored prior to and for four hours following oral dosage with captopril (one third daily dosage) and flosequinan (150 mg) on Day 1 and Day 2 of the study. The acute haemodynamic results of the study are summarised in Tables 1 and 2. The following abbreviations are used:

| | |
|---|---|
| PCWP | Pulmonary Capillary Wedge Pressure (mmHg) |
| CO | Cardiac Output (l/min) |
| CI | Cardiac Index (l/min/$m^2$) |
| SVR | Systemic Vascular Resistance (dyn/sec/$cm^{-5}$) |
| SVI | Stroke Volume Index (ml/beat/$cm^2$) |
| SWI | Stroke Work Index (g-m/$m^2$) |
| PVR | Pulmonary Vascular Resistance (dyn/sec/$cm^{-5}$) |
| HR | Heart Rate (beats/min) |
| MAP | Mean Arterial Pressure (mmHg) |
| RAP | Right Atrial Pressure (mmHg) |
| PAP Sys | Pulmonary Arterial Pressure - Systolic (mmHg) |
| PAP Dia | Pulmonary Arterial Pressure - Diastolic (mmHg) |
| BP Sys | Blood Pressure - Systolic (mmHg) |
| BP Dia | Blood Pressure - Diastolic (mmHg) |

Surprisingly, captopril and flosequinan, when co-administered, produced central haemodynamic effects indicative of improvement in the congestive heart failure of these very ill and unresponsive patients. At 24 hours an effect persisted on right atrial pressure (RAP) and pulmonary capillary wedge pressure (PCWP), indicators of pre-load, which can be attributed to flosequinan since the dose of captopril was stable.

Comparison of these data with the results of earlier studies in which 150 mg flosequinan was administered to 20 patients suffering from chronic congestive heart failure, suggests that the peak increase of cardiac output (CO) produced in this study was greater than that expected for flosequinan alone. This was particularly remarkable since no increase in heart rate (HR) was apparent. This implies that the increase in cardiac output (CO) evident after coadministration of captopril and flosequinan is due to an increase in stroke volume and not due, at least in part, to an increase in heart rate (HR) as expected.

Table 1

| Time after dosing (hours) | | PCWP | CO | CI | SVR | SVI | SWI | PVR |
|---|---|---|---|---|---|---|---|---|
| Day 1 | 0 (control) | 31.2 | 4.08 | 2.05 | 1641.9 | 27.5 | 20.2 | 268.4 |
| | 0.5 | 22.3 | 5.42 | 2.72 | 1144.0 | 36.1 | 25.6 | 234.9 |
| | 1.0 | 20.2 | 5.79 | 2.94 | 1027.4 | 36.3 | 28.8 | 253.9 |
| | 1.5 | 19.4 | 6.06 | 3.04 | 986.6 | 38.0 | 29.4 | 257.4 |
| | 2.0 | 21.7 | 5.84 | 3.00 | 978.2 | 37.9 | 27.8 | 212.7 |
| | 2.5 | 21.3 | 5.41 | 2.72 | 1164.9 | 36.7 | 27.6 | 263.3 |
| | 3.0 | 22.0 | 5.18 | 2.66 | 1164.9 | 36.6 | 27.5 | 256.5 |
| | 4.0 | 21.4 | 5.12 | 2.62 | 1275.5 | 33.4 | 28.4 | 288.7 |
| Day 2 | 0 (control) | 26.7 | 4.07 | 2.18 | 1546.9 | 28.4 | 22.5 | 306.1 |
| | 0.5 | 21.1 | 4.87 | 2.61 | 1172.3 | 34.0 | 25.2 | 265.6 |
| | 1.0 | 20.6 | 5.28 | 2.80 | 1047.1 | 36.2 | 26.4 | 243.9 |
| | 1.5 | 21.4 | 5.15 | 2.74 | 1114.2 | 34.7 | 26.7 | 254.1 |
| | 2.0 | 20.7 | 5.06 | 2.66 | 1161.5 | 35.9 | 27.3 | 267.7 |
| | 2.5 | 24.0 | 4.94 | 2.65 | 1244.8 | 33.8 | 25.4 | 235.1 |
| | 3.0 | 23.0 | 4.93 | 2.66 | 1235.7 | 35.2 | 28.1 | 267.4 |
| | 4.0 | 24.9 | 4.79 | 2.58 | 1327.0 | 32.0 | 26.5 | 279.2 |

Table 2

| Time after dosing (hours) | | HR | MAP | RAP | PAP Sys | PAP Dia | BP Sys | BP Dia |
|---|---|---|---|---|---|---|---|---|
| Day 1 | 0 (control) | 81.8 | 88.0 | 13.2 | 63.1 | 30.7 | 120.8 | 68.1 |
| | 0.5 | 80.9 | 77.0 | 10.2 | 56.8 | 25.8 | 111.9 | 58.1 |
| | 1.0 | 81.4 | 78.6 | 10.0 | 55.8 | 25.0 | 114.2 | 58.4 |
| | 1.5 | 82.8 | 77.2 | 9.6 | 54.2 | 24.1 | 112.9 | 57.1 |
| | 2.0 | 82.3 | 78.2 | 9.8 | 53.4 | 24.1 | 114.6 | 57.7 |
| | 2.5 | 80.4 | 79.8 | 9.2 | 52.8 | 24.4 | 115.7 | 59.7 |
| | 3.0 | 82.0 | 81.6 | 9.9 | 55.0 | 25.1 | 118.9 | 60.6 |
| | 4.0 | 81.3 | 84.7 | 9.2 | 56.8 | 24.9 | 125.0 | 61.9 |
| Day 2 | 0 (control) | 80.8 | 87.2 | 11.1 | 63.8 | 27.6 | 125.8 | 67.0 |
| | 0.5 | 81.8 | 77.4 | 9.0 | 57.2 | 24.0 | 116.4 | 58.0 |
| | 1.0 | 80.1 | 74.8 | 9.1 | 54.8 | 24.1 | 115.1 | 55.1 |
| | 1.5 | 81.0 | 78.0 | 9.6 | 57.4 | 24.7 | 118.6 | 58.6 |
| | 2.0 | 76.7 | 80.2 | 9.1 | 57.6 | 24.8 | 122.3 | 65.4 |
| | 2.5 | 80.7 | 81.2 | 10.1 | 57.6 | 25.2 | 122.7 | 61.7 |
| | 3.0 | 77.9 | 83.7 | 10.2 | 59.1 | 25.1 | 126.2 | 62.9 |
| | 4.0 | 83.8 | 84.7 | 10.1 | 60.4 | 27.7 | 130.0 | 64.7 |

## Claims

**Claims for the following Contracting States : AT, BE, CH, DE, DK, FR, GB, IT, LI, LU, NL, SE**

1. An angiotensin-converting enzyme (ACE) inhibitor and a compound of formula I

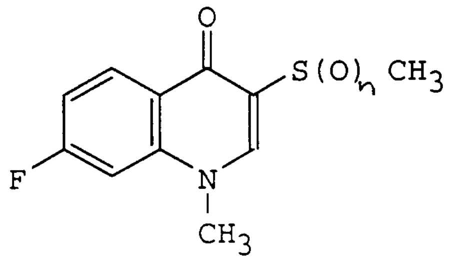

I

wherein n is 1 or 2, for simultaneous, separate or sequential use in the treatment of heart failure.

2. A product containing an angiotensin-converting enzyme (ACE) inhibitor and a compound of formula I

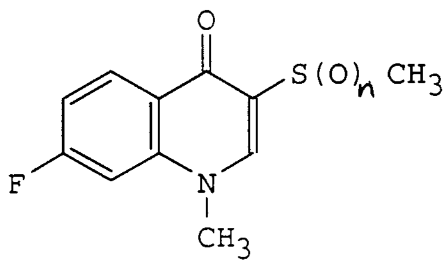

I

wherein n is 1 or 2, as a combined preparation for simultaneous, separate or sequential use for the treatment of heart failure.

3. A product as claimed in claim 2 wherein the ACE inhibitor is selected from the group consisting of captopril, enalapril, enalaprilat, quinapril, ramipril, cilazapril, delapril, fosenopril, zofenopril, indolapril, perindopril, spirapril, pentopril, pivopril, benazepril, benazeprilat, libenzapril and lisinopril.

4. A product as claimed in claim 3 wherein the ACE inhibitor is selected from the group consisting of captopril, enalapril and lisinopril.

5. A product as claimed in any one of claims 2 to 4 wherein the compound of formula I is 7-fluoro-1-methyl-3-methyl-sulphinyl-4-quinolone.

6. A pharmaceutical composition suitable for use in the treatment of heart failure comprising therapeutically effective amounts of an ACE inhibitor and a compound of formula I

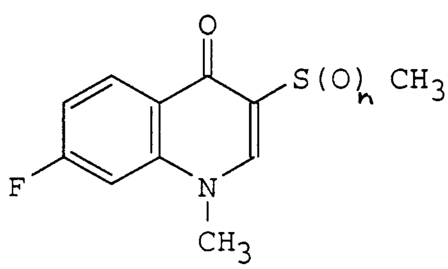

I

wherein n is 1 or 2, together with a pharmaceutically acceptable carrier.

**7.** A composition as claimed in claim 6 wherein the compound of formula I is 7-fluoro-1-methyl-3-methylsulphinyl-4-quinolone.

**8.** A composition as claimed in claim 6 or claim 7 wherein the ACE inhibitor is selected from the group consisting of captopril, enalapril, enalaprilat, quinapril, ramipril, cilazapril, delapril, fosenopril, zofenopril, indolapril, perindopril, spirapril, pentopril, pivopril, benazepril, benazeprilat, libenzapril and lisinopril.

**9.** A composition as claimed in claim 6 wherein the compound of formula I is 7-fluoro-1-methyl-3-methylsulphinyl-4-quinolone and the ACE inhibitor is selected from enalapril and lisinopril.

**10.** A composition as claimed in claim 9 in the form of a tablet.

**11.** The use of a compound of formula I

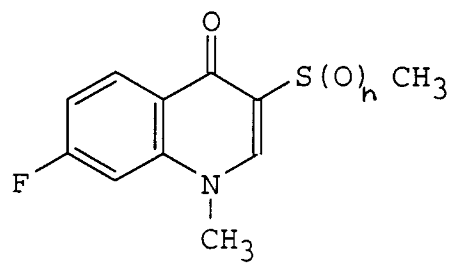

I

wherein n is 1 or 2 in the manufacture of a medicament for the treatment of heart failure in a patient who is also receiving treatment with an ACE inhibitor.

**Claims for the following Contracting State : ES**

**1.** An angiotensin-converting enzyme (ACE) inhibitor and a compound of formula I

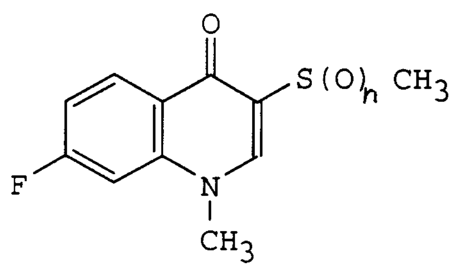

I

wherein n is 1 or 2, for simultaneous, separate or sequential use in the treatment of heart failure.

**2.** A method of making a product for use in the treatment of heart failure by combining an angiotensin-converting enzyme (ACE) inhibitor and a compound of formula I

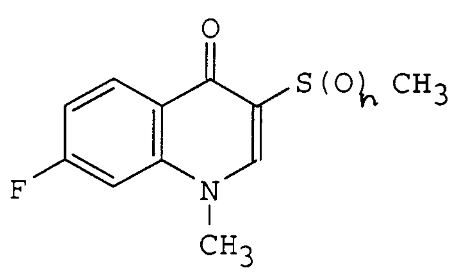

I

wherein n is 1 or 2, to provide a combined preparation for simultaneous, separate or sequential use.

3. A method as claimed in claim 2 wherein the ACE inhibitor is selected from the group consisting of captopril, enalapril, enalaprilat, quinapril, ramipril, cilazapril, delapril, fosenopril, zofenopril, indolapril, perindopril, spirapril, pentopril, pivopril, benazepril, benazeprilat, libenzapril and lisinopril.

4. A method as claimed in claim 3 wherein the ACE inhibitor is selected from the group consisting of captopril, enalapril and lisinopril.

5. A method as claimed in any one of claims 2 to 4 wherein the compound of formula I is 7-fluoro-1-methyl-3-methyl-sulphinyl-4-quinolone.

6. A method of preparing a pharmaceutical composition suitable for use in the treatment of heart failure by combining therapeutically effective amounts of an ACE inhibitor and a compound of formula I

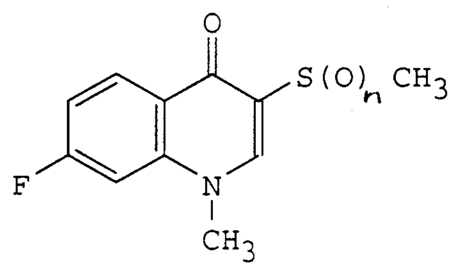

I

wherein n is 1 or 2, together with a pharmaceutically acceptable carrier.

7. A method as claimed in claim 6 wherein the compound of formula I is 7-fluoro-1-methyl-3-methyl-sulphinyl-4-quinolone.

8. A method as claimed in claim 6 or claim 7 wherein the ACE inhibitor is selected from the group consisting of captopril, enalapril, enalaprilat, quinapril, ramipril, cilazapril, delapril, fosenopril, zofenopril, indolapril, perindopril, spirapril, pentopril, pivopril, benazepril, benazeprilat, libenzapril and lisinopril.

9. A method as claimed in claim 6 wherein the compound of formula I is 7-fluoro-1-methyl-3-methyl-sulphinyl-4-quinolone and the ACE inhibitor is selected from enalapril and lisinopril.

10. A method as claimed in claim 9 wherein the composition is in the form of a tablet.

11. The use of a compound of formula I

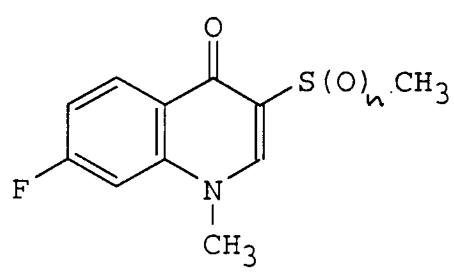

I

wherein n is 1 or 2 in the manufacture of a medicament for the treatment of heart failure in a patient who is also receiving treatment with an ACE inhibitor.

**Patentansprüche**

**Patentansprüche für folgende Vertragsstaaten : AT, BE, CH, DE, DK, FR, GB, IT, LI, LU, NL, SE**

1. Angiotensin-converting-enzyme (ACE) Hemmer sowie eine Verbindung der Formel I

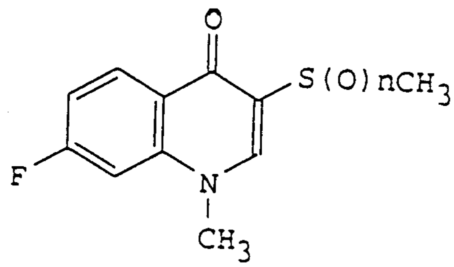

I ,

in der n 1 oder 2 ist, zur gleichzeitigen, getrennten oder aufeinanderfolgenden Verwendung bei der Behandlung von Herzversagen.

2. Produkt mit einem angiotensin-converting-enzyme (ACE) Hemmer und einer Verbindung der Formel I

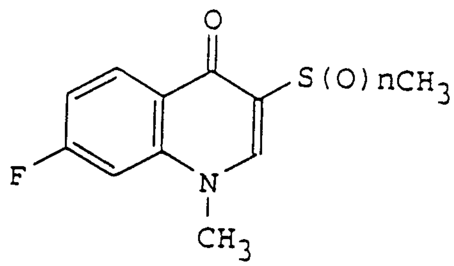

I ,

in der n 1 oder 2 ist, als Kombinationspräparat zur gleichzeitigen, getrennten oder aufeinanderfolgenden Verwendung für die Behandlung von Herzversagen.

3. Produkt nach Anspruch 2, in der der ACE-Hemmer aus der aus Captopril, Enalapril, Enalaprilat, Quinapril, Ramipril, Cilazapril, Delapril, Fosenopril, Zofenopril, Indolapril, Perindopril, Spirapril, Pentopril, Pivopril, Benazepril, Benazeprilat, Libenzapril und Lisinopril bestehenden Gruppe ausgewählt ist.

4. Produkt nach Anspruch 3, in dem der ACE-Hemmer aus der aus Captopril, Enalapril und Lisinopril bestehenden Gruppe ausgewählt ist.

5. Produkt nach einem der Ansprüche 2 bis 4, in dem die Verbindung der Formel I 7-Fluor-1-methyl-3-methyl-sulphinyl-4-chinolon ist.

6. Pharmazeutische Zubereitung, die zur Behandlung von Herzversagen geeignet ist und therapeutisch wirksame Mengen eines ACE-Hemmers und einer Verbindung der Formel I

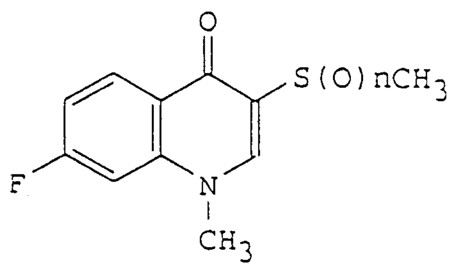

I ,

in der n 1 oder 2 ist, zusammen mit einem pharmazeutisch verträglichen Trägerstoff enthält.

7. Zubereitungung nach Anspruch 6, in der die Verbindung der Formel I 7-Fluor-1-methyl-3-methyl-sulphinyl-4-chinolon ist.

8. Zubereitung nach Anspruch 6 oder 7, in der der ACE-Hemmer aus der aus Captopril, Enalapril, Enalaprilat, Quinapril, Ramipril, Cilazapril, Delapril, Fosenopril, Zofenopril, Indolapril, Perindopril, Spirapril, Pentopril, Pivopril, Benazepril, Benazeprilat, Libenzapril und Lisinopril bestehenden Gruppe ausgewählt ist.

9. Zubereitung nach Anspruch 6, in der die Verbindung der Formel I 7-Fluor-1-methyl-3-methyl-sulphinyl-4-chinolon ist und der ACE-Hemmer aus Enalapril und Lisinopril ausgewählt ist.

10. Zubereitung nach Anspruch 9 in Form einer Tablette.

11. Verwendung einer Verbindung der Formel I

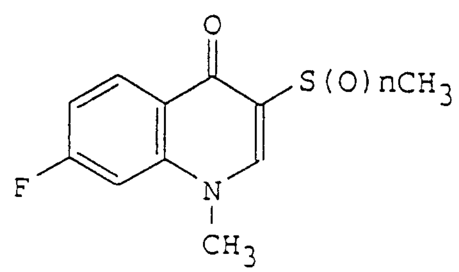

I,

in der n 1 oder 2 ist, für die Herstellung eines Medikaments für die Behandlung von Herzversagen bei einem Patienten, der auch mit einem ACE-Hemmer behandelt wird.

**Patentansprüche für folgenden Vertragsstaat : ES**

1. Angiotensin-converting-enzyme (ACE) Hemmer sowie eine Verbindung der Formel I

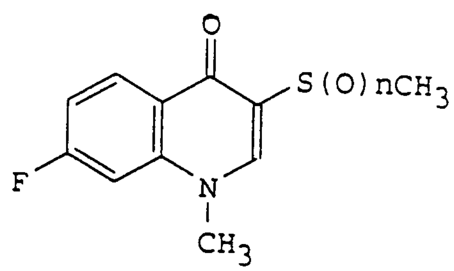

I,

in der n 1 oder 2 ist, zur gleichzeitigen, getrennten oder aufeinanderfolgenden Verwendung bei der Behandlung von Herzversagen.

2. Verfahren zur Herstellung eines Produktes zur Behandlung von Herzversagen durch Kombination eines angiotensin-converting-enzyme (ACE) Hemmers und einer Verbindung der Formel I

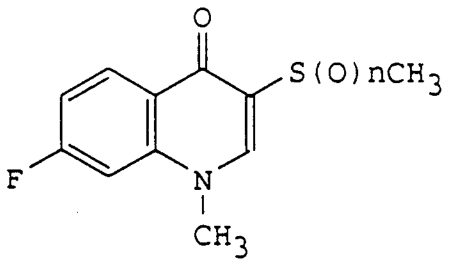

I ,

in der n 1 oder 2 ist, um ein Kombinationspräperat zur gleichzeitigen, getrennten oder aufeinanderfolgenden Verwendung für die Behandung zur Verfügung zu stellen.

**3.** Verfahren nach Anspruch 2, in der der ACE-Hemmer aus der aus Captopril, Enalapril, Enalaprilat, Quinapril, Ramipril, Cilazapril, Delapril, Fosenopril, Zofenopril, Indolapril, Perindopril, Spirapril, Pentopril, Pivopril, Benazepril, Benazeprilat, Libenzapril und Lisinopril bestehenden Gruppe ausgewählt ist.

**4.** Verfahren nach Anspruch 3, in dem der ACE-Hemmer aus der aus Captopril, Enalapril und Lisinopril bestehenden Gruppe ausgewählt ist.

**5.** Verfahren nach einem der Ansprüche 2 bis 4, in dem die Verbindung der Formel I 7-Fluor-1-methyl-3-methyl-sulphinyl-4-chinolon ist.

**6.** Verfahren zur Herstellung einer pharmazeutischen Zubereitung zur Behandlung von Herzversagen durch Kombination therapeutisch wirksamer Mengen eines ACE-Hemmers und einer Verbindung der Formel I

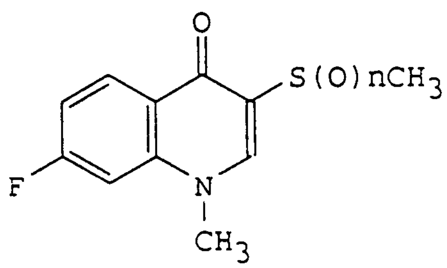

I ,

in der n 1 oder 2 ist, zusammen mit einem pharmazeutisch verträglichen Trägerstoff.

**7.** Verfahren nach Anspruch 6, in der die Verbindung der Formel I 7-Fluor-1-methyl-3-methyl-sulphinyl-4-chinolon ist.

**8.** Verfahren nach Anspruch 6 oder 7, in der der ACE-Hemmer aus der aus Captopril, Enalapril, Enalaprilat, Quinapril, Ramipril, Cilazapril, Delapril, Fosenopril, Zofenopril, Indolapril, Periondopril, Spirapril, Pentopril, Pivopril, Benazepril, Benazeprilat, Libenzapril und Lisinopril bestehenden Gruppe ausgewählt ist.

**9.** Verfahren nach Anspruch 6, in der die Verbindung der Formel I 7-Fluor-1-methyl-3-methyl-sulphinyl-4-chinolon ist und der ACE-Hemmer aus Enalpril und Lisinopril ausgewählt ist.

**10.** Verfahren nach Anspruch 9 in Form einer Tablette.

**11.** Verwendung einer Verbindung der Formel I

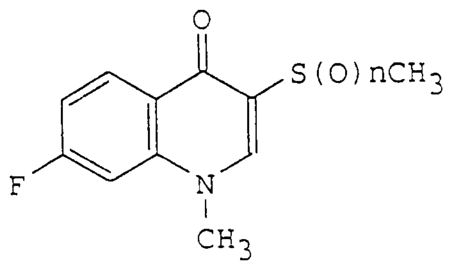

I ,

in der n 1 oder 2 ist, für die Herstellung eines Medikaments für die Behandlung von Herzversagen bei einem Patienten, der auch mit einem ACE-Hemmer behandelt wird.

**Revendications**
**Revendications pour les Etats contractants suivants : AT, BE, CH, DE, DK, FR, GB, IT, LI, LU, NL, SE**

**1.** Inhibiteur de l'enzyme de conversion d'angiotensine (ACE) et un composé de formule I

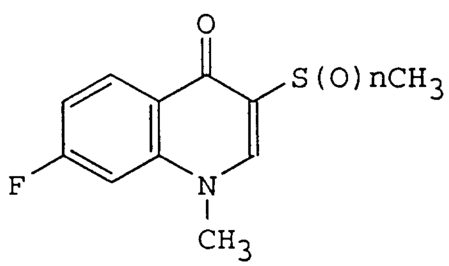

I

dans laquelle n est égal à 1 ou 2, destinés à être utilisés simultanément, séparément ou successivement dans le traitement de l'insuffisance cardiaque.

**2.** Produit contenant un inhibiteur de l'enzyme de conversion d'angiotensine (ACE) et un composé de formule I

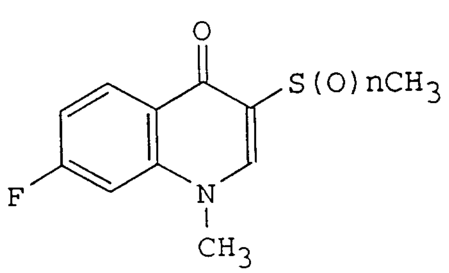

I

dans laquelle n est égal à 1 ou 2, sous forme d'une préparation mixte pour une utilisation simultanée, séparée ou successive pour le traitement de l'insuffisance cardiaque.

**3.** Produit suivant la revendication 2, dans lequel l'inhibiteur de ACE est choisi dans le groupe consistant en le captopril, l'énalapril, l'énalaprilat, le quinapril, le ramipril, le cilazapril, le délapril, le fosénopril, le zofénopril, l'indolapril, le périndopril, le spirapril, le pentopril, le pivopril, le bénazépril, le bénazéprilat, le libenzapril et le lisinopril.

**4.** Produit suivant la revendication 3, dans lequel l'inhibiteur de ACE est choisi dans le groupe consistant en le captopril, l'énalapril et le lisinopril.

5. Produit suivant l'une quelconque des revendications 2 à 4, dans lequel le composé de formule I est la 7-fluoro-1-méthyl-3-méthyl-sulfinyl-4-quinolone.

6. Composition pharmaceutique pouvant être utilisée dans le traitement de l'insuffisance cardiaque, comprenant des quantités à effet thérapeutique d'un inhibiteur de ACE et d'un composé de formule I

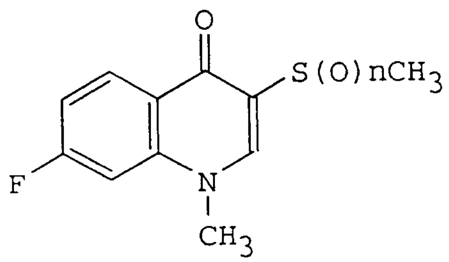

I

dans laquelle n est égal à 1 ou 2, en association avec un support pharmaceutiquement acceptable.

7. Composition suivant la revendication 6, dans laquelle le composé de formule I est la 7-fluoro-1-méthyl-3-méthyl-sulfinyl-4-quinolone.

8. Composition suivant la revendication 6 ou la revendication 7, dans laquelle l'inhibiteur de ACE est choisi dans le groupe consistant en le captopril, l'énalapril, l'énalaprilat, le quinapril, le ramipril, le cilazapril, le délapril, le fosénopril, le zofénopril, l'indolapril, le périndopril, le spirapril, le pentopril, le pivopril, le bénazépril, le bénazéprilat, le libenzapril et le lisinopril.

9. Composition suivant la revendication 6, dans laquelle le composé de formule I est la 7-fluoro-1-méthyl-3-méthylsulfinyl-4-quinolone et l'inhibiteur de ACE est choisi entre l'énalapril et le lisinopril.

10. Composition suivant la revendication 9, sous forme d'un comprimé.

11. Utilisation d'un composé de formule I

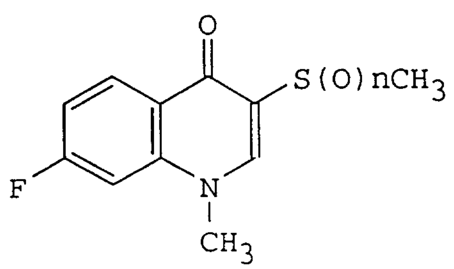

I

dans laquelle n est égal à 1 ou 2 dans la production d'un médicament destiné au traitement de l'insuffisance cardiaque chez un patient qui reçoit également un traitement avec un inhibiteur de ACE.

**Revendications pour l'Etat contractant suivant : ES**

1. Inhibiteur de l'enzyme de conversion d'angiotensine (ACE) et un composé de formule I

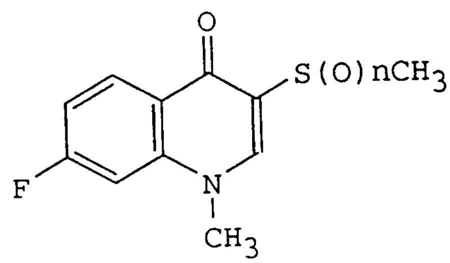

I

dans laquelle n est égal à 1 ou 2, destinés à être utilisés simultanément, séparément ou successivement dans le traitement de l'insuffisance cardiaque.

2. Procédé de production d'un produit destiné à être utilisé dans le traitement de l'insuffisance cardiaque par association d'un inhibiteur de l'enzyme de conversion d'angiotensine (ACE) et d'un composé de formule I

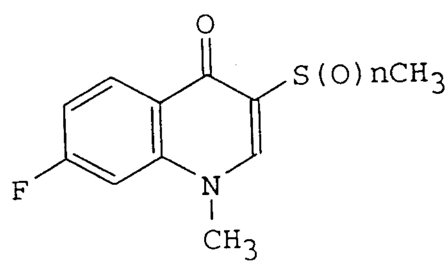

I

dans laquelle n est égal à 1 ou 2, pour obtenir une préparation mixte pour une utilisation simultanée, séparée ou successive.

3. Procédé suivant la revendication 2, dans lequel l'inhibiteur de ACE est choisi dans le groupe consistant en le captopril, l'énalapril, l'énalaprilat, le quinapril, le ramipril, le cilazapril, le délapril, le fosénopril, le zofénopril, l'indolapril, le périndopril, le spirapril, le pentopril, le pivopril, le bénazépril, le bénazéprilat, le libenzapril et le lisinopril.

4. Procédé suivant la revendication 3, dans lequel l'inhibiteur de ACE est choisi dans le groupe consistant en le captopril, l'énalapril et le lisinopril.

5. Procédé suivant l'une quelconque des revendications 2 à 4, dans lequel le composé de formule I est la 7-fluoro-1-méthyl-3-méthyl-sulfinyl-4-quinolone.

6. Procédé de préparation d'une composition pharmaceutique pouvant être utilisée dans le traitement de l'insuffisance cardiaque par association de quantités à effet thérapeutique d'un inhibiteur de ACE et d'un composé de formule I

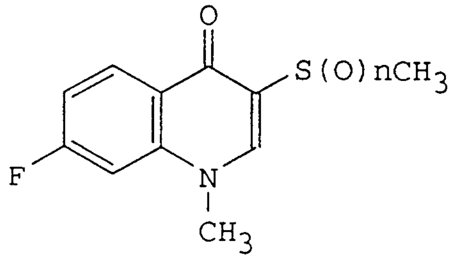

I

dans laquelle n est égal à 1 ou 2, conjointement avec un support pharmaceutiquement acceptable.

7. Procédé suivant la revendication 6, dans lequel le composé de formule I est la 7-fluoro-1-méthyl-3-méthyl-sulfinyl-4-quinolone.

8. Procédé suivant la revendication 6 ou la revendication 7, dans lequel l'inhibiteur de ACE est choisi dans le groupe consistant en le captopril, l'énalapril, l'énalaprilat, le quinapril, le ramipril, le cilazapril, le délapril, le fosénopril, le zofénopril, l'indolapril, le périndopril, le spirapril, le pentopril, le pivopril, le bénazépril, le bénazéprilat, le libenzapril et le lisinopril.

9. Procédé suivant la revendication 6, dans lequel le composé de formule I est la 7-fluoro-1-méthyl-3-méthylsulfinyl-4-quinolone et l'inhibiteur de ACE est choisi entre l'énalapril et le lisinopril.

10. Procédé suivant la revendication 9, dans lequel la composition est sous forme d'un comprimé.

11. Utilisation d'un composé de formule I

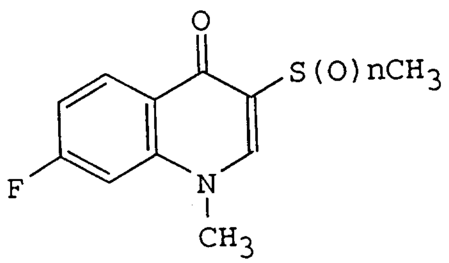

I

dans laquelle n est égal à 1 ou 2 dans la production d'un médicament destiné au traitement de l'insuffisance cardiaque chez un patient recevant également un traitement avec un inhibiteur de ACE.